(19) Europäisches Patentamt
European Patent Office
Office européen des brevets

(11) Veröffentlichungsnummer: **0 201 103 B1**

(12) **EUROPÄISCHE PATENTSCHRIFT**

(45) Veröffentlichungstag der Patentschrift: **10.04.91**

(51) Int. Cl.$^5$: **A61B 17/39**

(21) Anmeldenummer: **86106305.5**

(22) Anmeldetag: **07.05.86**

# (54) Überwachungsvorrichtung für ein Hochfrequenz-Chirurgiegerät.

(30) Priorität: **07.05.85 DE 3516354**

(43) Veröffentlichungstag der Anmeldung:
**12.11.86 Patentblatt 86/46**

(45) Bekanntmachung des Hinweises auf die Patenterteilung:
**10.04.91 Patentblatt 91/15**

(84) Benannte Vertragsstaaten:
**DE FR GB IT**

(56) Entgegenhaltungen:
**DE-A- 3 239 640**

(73) Patentinhaber: **Irnich, Werner, Prof. Dr.-Ing.**
**Birkenweg 60**
**W-6301 Wettenberg 3(DE)**

(72) Erfinder: **Irnich, Werner, Prof. Dr.-Ing.**
**Birkenweg 60**
**W-6301 Wettenberg 3(DE)**

(74) Vertreter: **Biermann, Wilhelm, Dr.-Ing.**
**Morillenhang 39**
**W-5100 Aachen(DE)**

**EP 0 201 103 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft eine Überwachungsvorrichtung für ein Hochfrequenz-Chirurgiegerät, mit einer in größerer Entfernung zur aktiven Elektrode am Körper angebrachten, während der Behandlung die auf der Körperoberfläche bestehende auf das Potential der neutralen Elektrode bezogene Hochfrequenzspannung abgreifenden Kontrollelektrode und mit einer von der Kontrollelektrode angesteuerten, einen Spannungskomparator umfassenden Überwachungsschaltung, wobei der Spannungskomparator eine von der Körperoberflächenspannung abhängige Spannung mit einer vorgegebenen Referenzspannung vergleicht und bei Überschreiten der Referenzspannung einen Signalgeber und/oder ein das Hochfrequenz-Chirurgiegerät abschaltendes Relais ansteuert.

Bei einer bekannten Überwachungsvorrichtung dieser Art (DE-A-33 06 402) wird die von der Kontrollelektrode abgegriffene Spannung zunächst einer Quadrierstufe, und die quadrierte Spannung einer Integrierstufe zugeführt, und die quadrierte und integrierte Spannung wird in der Komparatorschaltung mit einer Referenzspannung verglichen. Bei dieser bekannten Überwachungsvorrichtung wird davon ausgegangen, daß die einwirkende elektrische Gesamtenergie, die sich aus dem Produkt der elektrischen Feldstärke und der Stromdichte einerseits und aus der Einwirkungszeit andererseits ergibt, ein Maß für die Verbrennungsgefahr darstellt.

Es hat sich gezeigt, daß auch bei gleicher zugeführter elektrischer Gesamtenergie, und auch bei ordnungsgemäßem Kontakt der neutralen Elektrode zur Körperoberfläche, die effektive Hitzeentwicklung an der am meisten belasteten Körperstelle unter der neutralen Elektrode bei den einzelnen Patienten unterschiedlich ist.

Der Erfindung liegt deshalb die Aufgabe zugrunde, eine Überwachungsvorrichtung für ein HF-Chirurgiegerät der eingangs genannten Art zu schaffen, die trotz der individuell unterschiedlichen Hitzeentwicklung bei den einzelnen Patienten die Ge fahr einer Verbrennung mit Sicherheit ausschaltet.

Gemäß der Erfindung wird diese Aufgabe dadurch gelöst, daß dem Spannungskomparator einerseits eine der von der Kontrollelektrode abgegriffenen Spannung direkt proportionale Spannung, und andererseits als Referenzspannung eine einer zuvor bei jedem Patienten individuell ermittelten maximal erträglichen Körperoberflächenspannung entsprechende Spannung zugeführt wird.

Die beobachtete individuell unterschiedliche Hitzeentwicklung bei den einzelnen Patienten läßt sich dadurch erklären, daß zum einen die elektrische Leitfähigkeit der Haut infolge unterschiedlicher Dicke und unterschiedlicher Feuchtigkeit der Haut verschieden ist, und daß zum anderen auch das Vermögen des Blutkreislaufs, die elekrisch eingebrachte Wärme wegzutransportieren, individuell unterschiedlich ist. Auf dieser Erkenntnis aufbauend schlägt die Erfindung vor, als Kriterium für die Gefahr einer Verbrennung die von der Kontrollelektrode abgegriffene Körperoberflächenspannung selbst, bzw. eine hierzu direkt proportionale Spannung, zu benutzen, wobei diese mit der maximal erträglichen Körperoberflächenspannung verglichen wird, die für jeden Patienten individuell vorher zu ermitteln ist.

Die bei jedem einzelnen Patienten maximal erträgliche Körperoberflächenspannung, und damit die Referenzspannung für den Spannungskomparator, läßt sich auf verschiedene Weise, beispielsweise mit Hilfe des HF-Chirurgiegerätes und der Kontrollelektrode selbst, durch eine einmalige Messung vor der Operation bestimmen. Zu diesen Zweck werden die neutrale Elektrode und die Kontrollelektrode angelegt und über eine weitere großflächige Elektrode die HF-Spannung zugeführt und langsam erhöht, bis der Patient subjektiv ein deutliches Wärmegefühl unter der neutralen Elektrode empfindet.

Zweckmäßigerweise erfolgt jedoch die individuelle Bestimmung der im Einzelfall maximal erträglichen Körperoberflächenspannung mit Hilfe einer separaten Vorrichtung, die zwei in einem festen Abstand voneinander angeordnete Kontaktelektroden und einen eine einstellbare HF-Spannung liefernden HF-Generator umfaßt. Auf diese Weise läßt sich die Bestimmung der maximalen Oberflächenspannung und damit der im Einzelfall einzustellenden Referenzspannung vereinfachen und schneller durchführen. Als Kriterium für die maximale Oberflächenspannung kann auch in diesem Fall die subjektive Schmerzempfindung des Patienten dienen. Statt dessen kann jedoch vorteilhafterweise auch eine objektive Temperaturmessung vorgenommen werden, indem beispielsweise in einer oder in beiden Kontaktelektroden ein Temperaturfühler angeordnet wird, der die Hauttemperatur mißt. Als Anhaltswert für die maximal tolerable Temperatur auf der Haut gilt dabei eine Temperatur von etwa 43 bis 44 °C. Falls eine separate Meßvorrichtung mit zwei kleinflächigen Kontaktelektroden verwendet wird, bei der die Kontaktfläche der Elektroden jeweils etwa 1 bis 2 cm$^2$ beträgt und die Elektroden nicht zu weit voneinander entfernt sind, dann gilt, daß die Spannung zwischen den beiden Kontaktelektroden bei maximal tolerabler Spannung etwa doppelt so hoch ist wie die maximal tolerable Körperoberflächenspannung bei Anwendung des HF-Chirurgiegerätes.

In zweckmäßiger Weiterbildung der Erfindung umfaßt die Überwachungsvorrichtung einen weiteren Spannungskomparator, dem einerseits eine der von der Kontrollelektrode abgegriffenen Spannung direkt proportionale Spannung, und andererseits als Referenzspannung eine zu der Arbeitsspannung direkt proportionale Spannung zugeführt und das Verhältnis der Spannungen so gewählt wird, daß der Spannungskomparator ein Signal gibt, wenn die Körperoberflächenspannung einen Wert von etwa 10 % der Arbeitsspannung übersteigt. Auf diese Weise läßt sich zusätzlich überprüfen, ob die neutrale Elektrode ordnungsgemäß angelegt ist und einen guten, großflächigen Kontakt zur Körperoberfläche hat.

Die zuletzt genannte Maßnahme geht von der Überlegung aus, daß unter der Voraussetzung, daß bei ordnungsgemäßem Kontakt der neutralen Elektrode der die aktive Elektrode durchfließende Strom und der die neutrale Elektrode durchfließende Strom identisch sind, die Körperoberflächenspannung dividiert durch die Übergangsimpedanz der neutralen Elektrode gleich der Gesamtspannung dividiert durch die Gesamtimpedanz des Stromkreises ist. Aus dieser Beziehung kann man durch Umformung ableiten, daß das Verhältnis der Körperoberflächenspannung zur Gesamtspannung gleich dem Verhältnis der Impedanz der neutralen Elektrode zur Gesamtimpedanz ist. Bei ordnungsgemäßem Kontakt der neutralen Elektrode sollte dieses Verhältnis jedoch nicht größer las etwa 1 : 10 sein. Infolgedessen kann durch eine Überprüfung der Körperoberflächenspannung unter dem Gesichtspunkt, ob sie etwa 1/10 der angelegten Spannung übersteigt, eine unzulässige Erhöhung der Übergangsimpedanz an der neutralen Elektrode festgestellt werden.

Das Impedanzverhältnis ist andererseits in jedem Fall größer als etwa 3 : 100. Diese Tatsache kann in weiterer Ausgestaltung der Erfindung dazu genutzt werden, mit Hilfe eines weiteren Spannungskomparators zu überwachen, ob die Kontrollelektrode selbst guten Kontakt zur Körperoberfläche hat oder ob sie sich unter Umständen gelöst hat. Zu diesem Zweck wird also dem weiteren Spannungskomparator einerseits die von der Kontrollelektrode abgegriffene Körperoberflächenspannung und andererseits eine zu der Arbeitsspannung direkt proportionale Spannung zugeführt und das Verhältnis der Spannungen so gewählt, daß der Spannungskomparator ein Signal gibt, wenn die von der Kontrollelektrode abgegriffene Spannung kleiner ist als etwa 3 % der Arbeitsspannung.

Schließlich kann die erfindungsgemäße Überwachungsvorrichtung durch einen weiteren Spannungskomparator ergänzt werden, dem als Referenzspannung eine bestimmte konstante Spannung zugeführt wird und der ein Signal gibt, wenn die von der Kontroll elektrode abgegriffene Oberflächenspannung Spannungsspitzen von etwa 150 V erreicht oder überschreitet. Mit Hilfe einer solchen Überwachung der Spannungsspitzen lassen sich diejenigen Verbrennungen oder auch Funkendurchschläge durch die Haut vermeiden, die aufgrund von Spannungsspitzen bei gegebenenfalls nur kurzzeitigen Kontaktverschlechterungen der neutralen Elektrode auftreten können. Da in diesem Fall die Verbrennungsgefahr besonders groß ist, muß das Elektrochirurgiegerät durch das vom Spannungskomparator gegebene Signal sofort abgeschaltet werden.

Weitere Merkmale und Vorteile der Erfindung ergeben sich aus der nachfolgenden Beschreibung anhand der Zeichnungen. Von den Zeichnungen zeigt

- Fig. 1 den Aufbau einer erfindungsgemäßen Überwachungsvorrichtung als Blockschaltbild;
- Fig. 2 eine separate Vorrichtung zum individuellen Bestimmen der maximal erträglichen Körperoberflächenspannung eines Patienten;
- Fig. 3 den Aufbau eines in der Überwachungsvorrichtung verwendeten Spannungsumformers;
- Fig. 4 den Aufbau einer erfindungsgemäßen Überwachungsvorrichtung mit zusätzlichen Kontrollfunktionen, in Form eines Blockschaltbildes; und
- Fig. 5 das Schaltbild eines in der Überwachungsvorrichtung verwendbaren Spitzenwertdetektors.

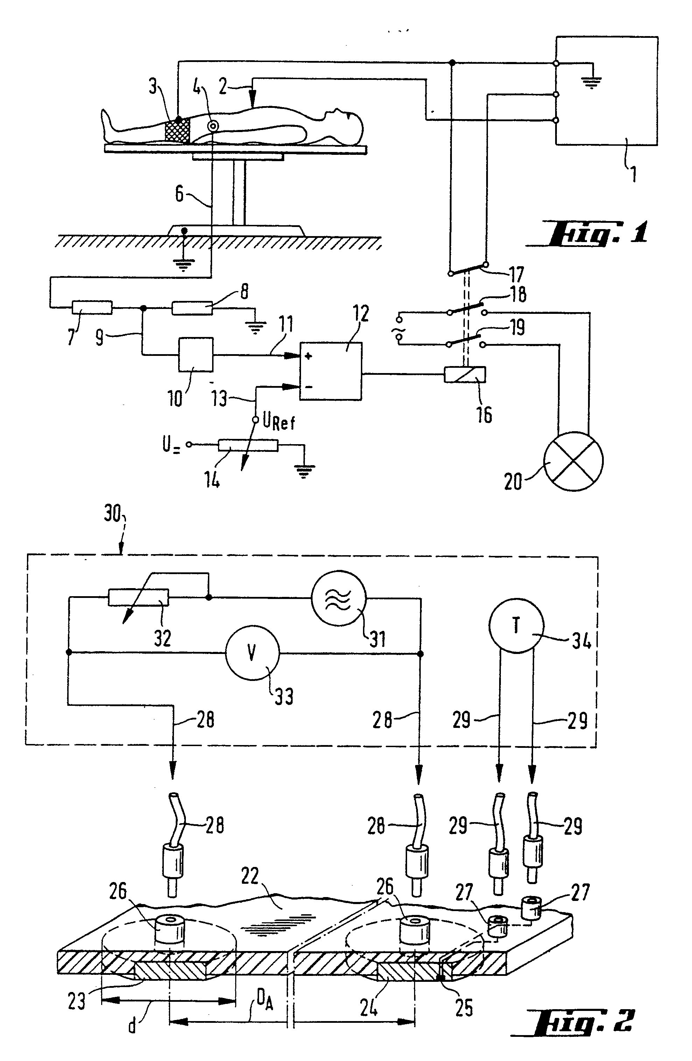

Wie aus Fig. 1 ersichtlich ist, sind an das Elektrochirurgiegerät 1 die aktive Elektrode 2 und die neutrale Elektrode 3 angeschlossen. Die neutrale Elektrode 3 ist großflächig ausgebildet und beispielsweise am Oberschenkel des Patienten angelegt und weist Erdpotential auf.

Mit Hilfe der auf der Körperoberfläche des Patienten angeordneten Überwachungselektrode 4, die weder die neutrale Elektrode 3 noch ein anderes geerdetes Teil berühren darf, wird die gegenüber dem Erdpotential bestehende Körperoberflächenspannung abgegriffen. Diese auf der Körperoberfläche bestehende Spannung ist weitgehend unabhängig vom Angriffspunkt der verschiedenen Elektroden, so daß die Lage der Kontrollelektrode 4 auf der Körperoberfläche nicht kritisch ist.

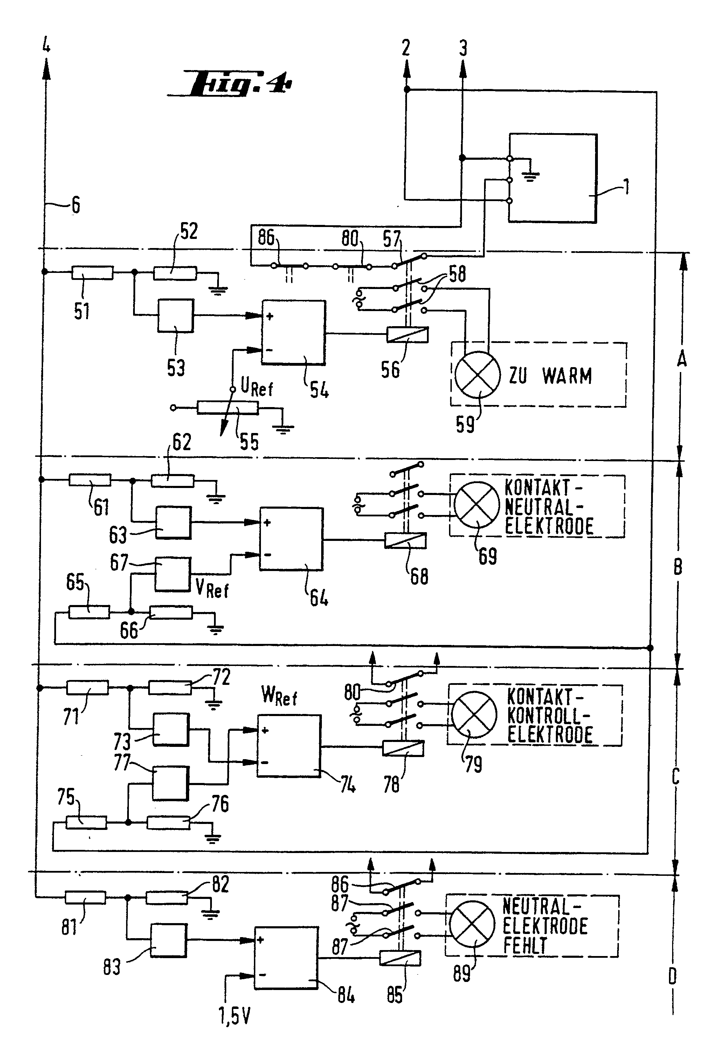

Die von der Kontrollelktrode 4 abgegriffene Körperoberflächenspannung wird über die Leitung 6 einem aus den Widerständen 7 und 8 bestehenden Spannungsteiler zugeführt und so weit geteilt, daß hinter dem Widerstand 7 für die weitere Verarbeitung eine Spannung geeigneter Größe zur Verfügung steht. Diese heruntergeteilte Hochfrequenzspannung wird über die Leitung 9 einem Umformer 10 zugeführt, in dem die Hochfrequenzspannung in eine dem Effektivwert der HF-Spannung entsprechende Gleichspannung umgewandelt wird. Diese Gleichspannung wird über die Leitung 11 dem Plus-Eingang eines Komparators 12 zugeführt. Dem Minus-Eingang dieses Komparators 12 wird über die Leitung 13 eine Referenzspannung $U_{Ref}$ zugeführt, und der Komparator 12 vergleicht die über die Leitung 11 zugeführte Spannung mit dieser Referenzspannung $U_{Ref}$. Die Referenzspannung $U_{Ref}$ wird mit Hilfe eines Potentiometers 14 erhalten, das von einer konstanten Gleichspannung $U_=$ versorgt wird. Diese Referenzspannung $U_{Ref}$ wird für jeden Patienten individuell eingestellt, nachdem zuvor beispielsweise mit der anhand der Fig. 2 beschriebenen Vorrichtung festgestellt worden ist, wie hoch bei dem betreffenden Patienten die Referenzspannung $U_{Ref}$ zu wählen ist.

Wenn die von dem Umformer 10 dem Komparator 12 zugeführte Spannung gleich oder höher ist als die Referenzspannung $U_{Ref}$, wird von dem Komparator 12 ein Relais 16 angesteuert.

Dieses Relais 16 schaltet über den Kontakt 17 das Hochfrequenz-Chirurgiegerät 1 ab und schaltet gleichzeitig über die Kontakte 18, 19 einen Signalgeber 20 ein, der ein optisches und/oder ein akustisches Signal gibt.

Zur Bestimmung der Höhe der am Potentiometer 14 einzustellenden Referenzspannung $U_{Ref}$ bedient man sich zweckmäßig einer Meßvorrichtung, wie sie anhand der Fig. 2 beschrieben wird.

Diese Meßvorrichtung besteht aus einer Trägerplatte 22 aus elektrisch isolierendem Material, in der zwei beispielsweise kreisförmige Kontaktelektroden 23, 24 so angeordnet sind, daß sie durch Auflegen der Trägerplatte 22 auf ein Körperteil des Patienten mit der Haut des Patienten in Kontakt gebracht werden. Die Kontaktfläche der Elektroden 23, 24 beträgt größenordnungsmäßig jeweils etwa 1 bis 2 $cm^2$, das heißt ihr Durchmesser d beträgt jeweils etwa 1 bis 1,6 cm. Der Abstand $D_A$ der beiden Elektroden 23, 24 voneinander beträgt etwa das Zehnfache ihres Durchmessers d, also etwa 10 bis 16 cm. In einer der Elektroden 23, 24, oder gegebenenfalls in beiden Elektroden, ist ein Temperaturfühler 25 integriert, der es gestattet, die Temperatur der Hautoberfläche zu messen.

Die Trägerplatte 22 ist auf der der Kontaktseite gegenüber liegenden Oberfläche mit Anschlußbuchsen 26 für die Kontaktelektroden 23, 24, und mit Anschlußbuchsen 27 für den Thermofühler 25 versehen. Über diese Anschlußbuchsen 26, 27 wird die elektrische Verbindung zu einem Gerät 30 hergestellt. Dieses Gerät 30, dessen Aufbau schematisch dargestellt ist, weist einerseits einen HF-Generator 31, ein Potentiometer 32 und ein Voltmeter 33 zur Erzeugung und Kontrolle eines einstellbaren HF-Stroms, und andererseits ein Anzeigeinstrument 34 auf. Die HF-Spannung wird den beiden Elektroden 23, 24 über die Leitungen 28 zugeführt, und das Anzeigeinstrument 34 wird über die Leitungen 29 mit den Anschlußbuchsen 27 verbunden. Das Meßinstrument 34 dient zur Anzeige der Hauttemperatur.

Mit Hilfe des Potentiometers 32 läßt sich die HF-Spannung in dem benötigten Ausmaß derart verändern, daß bei einer Kontaktfläche einer Elektrode von etwa 1 $cm^2$ durch den Stromkreis ein Strom zwischen etwa 5 und 20 mA fließt.

Zur Bestimmung der Referenzspannung $U_{Ref}$ wird also die Trägerplatte 22 an einer geeigneten Stelle mit der Haut des Patienten in Kontakt gebracht, und es werden die elektrischen Verbindungen zu dem Gerät 30 hergestellt. Sodann wird mit Hilfe des Potentiometers 32 die HF-Spannung zwischen den beiden Elektroden langsam erhöht, bis der Patient eine gerade noch tolerierbare Erwärmung unter den Elektroden feststellt. Die maximal tolerierbare Erwärmung liegt in der Regel dann vor, wenn der Thermofühler 25 eine Hautoberflächentemperatur von etwa 43 bis 44 °C feststellt. Wenn also das Meßinstrument 34 diese Temperatur anzeigt, wird der Effektivwert dieser HF-Spannung am Voltmeter 33 abgelesen. Die abgelesene Spannung stellt den doppelten Wert der bei der Anordnung nach Fig. 1 maximal tolerierbaren Spannung dar, weil bei der beschriebenen Meßvorrichtung die Spannung über zwei Elektroden zugeführt wird. Um die Referenzspannung $U_{Ref}$ zu erhalten, die am Potentiometer 14 einzustellen ist, muß also der am Voltmeter 33 abgelesene Effektivwert der HF-Spannung halbiert werden.

In Fig. 3 ist der beispielsweise Aufbau eines Umformers 10 schematisch dargestellt, mit dem bei der anhand der in Fig. 1 beschriebenen Überwachungsschaltung die mit der Kontrollelektrode 4 abgegriffene HF-Spannung in eine entsprechende Gleichspannung umgeformt wird, die als solche mit der ermittelten Referenzspannung $U_{Ref}$ verglichen werden kann. Bei dem dargestellten Umformer handelt es sich um einen als solchen bekannten sogenannten linearisierten Thermoumformer. Ein Thermoumformer, wie er zur Messung von HF-Strömen benutzt wird, besteht bekanntlich aus einen Heizdraht, der durch den zu messenden Strom erhitzt wird, und einem von diesem Heizdraht erhitzten Thermoelement, dessen Thermospannung als Maß für die Größe des HF-Stroms dient. Sei dem hier zur Anwendung kommenden "linearisierten Thermoumformer" ist ein Thermoumformer 38, der einen Heizdraht 39 und ein Thermoelement 40 aufweist, mit einem gleichen, einen Heizdraht 42 und ein Thermoelement 43 aufweisenden Thermoumformer 41 in Subtraktionsschaltung verbunden, wobei die Minus-Ausgänge der beiden Thermoumformer unmittelbar miteinander verbunden werden und die Plus-Ausgänge der beiden Thermoumformer zu einem Operationsverstärker 45 führen. Dabei ist der Plus-Ausgang dem Thermoelements 40 mit dem Plus-Eingang des Operationsverstärkers 45, und der Plus-Ausgang des Thermoelements 43 mit dem Minus-Eingang des Operationsverstärkers 45 verbunden. Wird nun im Heizdraht 39, zu dem über die Leitung 9 die entsprechend heruntergeteilte Körperoberflächenspannung zugeführt wird, durch, den Strcm $I_1$ Wärme erzeugt, dann ergibt sich am Plus-Eingang des Verstärkers 45 eine Spannung, die mit der Verstärkung V des Verstärkers 45 am Ausgang des Verstärkers 45 erscheint. Diese Ausgangsspannung, die am Punkt 46 abgegriffen werden kann, bewirkt ihrerseits einen Stromfluß $I_2$ durch den Widerstand 42 des Thermoumformers 41 und damit eine entsprechende Erwärmung dieses Widerstandes 42, die ihrerseits zu einer Spannung am Thermoelement 43 führt. Der Regelkreis sorgt dafür, daß an den Widerständen 39 und 42 stets die gleiche Wärme erzeugt wird, und daß damit die von dem Verstärker 45 gelieferte Ausgangsspannung, die über die Leitung 11 dem Komparator zugeführt wird, stets gleich dem Effektivwert der über die Leitung 9 zugeführten HF-Spannung ist.

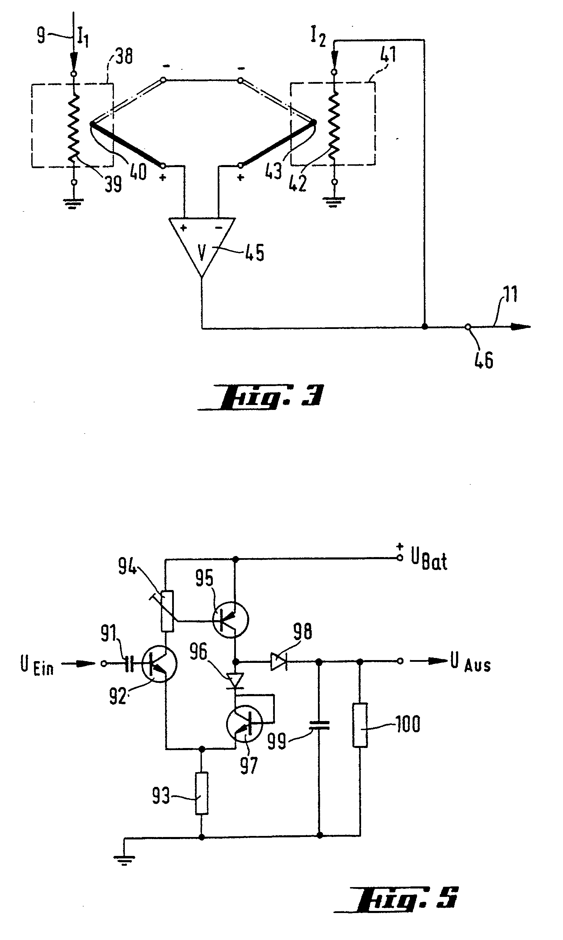

In Fig. 4 ist eine Überwachungsvorrichtung in ihrem schaltungsmäßigen Aufbau dargestellt, die nicht nur die Gefahr einer unzulässig hohen Erwärmung vermeidet, sondern die darüber hinaus auf dem gleichen Meßprinzip aufbauend auch andere Gefahren und Fehler zu erkennen gestattet. Die Überwachungsvorrichtung wird von der Kontrollelektrode 4 über die Leitung 6 angesteuert, und wirkt u.a. unmittelbar auf das Elektrochirurgiegerät 1 ein, zu dem die neutrale Elektrode 3 und die aktive Elektrode 2 führen. Die dargestellte Schaltung besteht aus vier Teilen, nämlich dem Teil A, dem Teil B, dem Teil C und dem Teil D.

Der Teil A entspricht der in Fig. 1 dargestellten Vorrichtung und dient zur Kontrolle der Hauterwärmung. Die von der Körperoberfläche abgegriffene HF-Spannung wird zunächst im Spannungsteiler 51, 52 auf den zehnten Teil heruntergeteilt und dann einem linearisierten Thermoumformer 53 zugeführt. Die am Ausgang des Thermoumformers 53 entstehende Gleichspannung wird im Komparator 54 mit einer Referenzspannung $U_{Ref}$ verglichen, die zuvor patientenindividuell ermittelt und dementsprechend an dem Potentiometer 55 eingestellt wurde. Sobald die Körperoberflächenspannung die Referenzspannung $U_{Ref}$ überschreitet, wird von dem Komparator 54 das Relais 56 angesteuert. Das Relais 56 ist zweckmäßigerweise so ausgebildet, daß es um etwa 1 s anzugsverzögert und über einen Zeitraum von 30 bis 60 s selbsthaltend arbeitet. In diesem Fall wird das Elektrochirurgiegerät 1 über den Schaltkontakt 57 nur für den Zeitraum von 30 bis 60 s ausgeschaltet, währenddessen eine Abkühlung der erwärmten Hautbereiche erfolgt. Danach schaltet das Relais 56 das Elektrochirurgiegerät 1 wiederum für einen Zeitraum von 1 s ein, so daß gewissermaßen intermittierend weiter operiert werden kann und eine Verbrennungsgefahr ausgeschlossen ist. Über die Kontakte 58 des Relais 56 wird dieser eingetretene Fall der Gefahr einer Überhitzung der Haut durch ein Lichtsignal 59 erkennbar gemacht.

Der Teil B der Überwachungsschaltung dient dazu, die neutrale Elektrode 3 daraufhin zu überprüfen, ob sie guten Kontakt zur Körperoberfläche des Patienten hat. Die von der Kontrollelektrode 4 abgegriffene und über die Leitung 6 ankommende Körperoberflächenspannung wird wiederum in einem Spannungsteiler 61, 62 auf den zehnten Teil heruntergeteilt und einem HF-Umformer 63 zugeführt. bei dem HF-Umformer 63 handelt es sich in diesem Fall um einen Spitzenwertdetektor, dessen Ausgangs-Gleichspannung dem Spitzenwert der zugeführten HF-Spannung entspricht. Die Ausgangs-Gleichspannung des Umsetzers 63 wird dem Komparator 64 zugeführt.

Als Referenzspannung wird dem Komparator 64 eine Spannung $V_{Ref}$ zugeführt, die aus der Arbeitsspannung des Chirurgiegeräts 1 abgeleitet wird. Zu diesem Zweck wird die an der Zuleitung zur aktiven Elektrode 2 abgegriffene Arbeitsspannung in einem Spannungsteiler mit den Widerständen 65 und 66 auf den hundertsten Teil heruntergeteilt. Die so reduzierte Spannung wird einem Umformer 67 zugeführt, bei dem es sich um einen linearisierten Thermoumformer handeln kann, der eine dem Effektivwert der HF-Eingangsspannung entsprechende Ausgangs-Gleichspannung liefert. Diese Ausgangs-Gleichspannung dient als Referenzspannung $V_{Ref}$ für den Komparator 64. Der Komparator 64 erzeugt an seinem Ausgang ein Signal, wenn die eingehenden Spannungen sich wie 1 : 10 verhalten, das heißt wenn ein Hundertstel der Arbeitsspannung gleich oder kleiner ist als ein Zehntel der Körperoberflächenspannung. In diesem Fall wird von dem Komparator 64 das Relais 68 angesteuert, das daraufhin anzieht und über seine Kontakte ein Licht- oder Tonsignal 69 erzeugt als Hinweis darauf, daß sich der Übergangskontakt der neutralen Elektrode verschlechtert hat.

Durch den Teil C der Überwachungsschaltung wird überprüft, ob die Kontrollelektrode 4 selbst einen einwandfreien Kontakt zur Körperoberfläche des Patienten hat. Die Körperoberflächenspannung wird in diesem Fall mit Hilfe des aus den beiden Widerständen 71 und 72 bestehenden Spannungsteilers auf ein Drittel heruntergeteilt und über den HF-Umformer 73, bei dem es sich um einen Spitzenwertdetektor handeln kann, wie er später anhand der Fig. 5 beschrieben wird, dem Minus-Eingang des Komparators 74 zugeleitet. Als Referenzspannung $W_{Ref}$ wird wiederum eine von der Arbeitsspannung des Elektrochirurgiegerätes 1 abgeleitete Spannung verwendet. Die von der Zuleitung zur aktiven Elektrode 2 abgegriffene Arbeitsspannung wird zu diesem Zweck in dem aus den Widerständen 75 und 76 bestehenden Spannungsteiler auf den hundertsten Teil heruntergeteilt und über den HF-Umformer 77, bei dem es sich wiederum um einen Spitzenwert-Detektor entsprechend Fig. 5 handeln kann, dem Plus-Eingang des Komparators 74 als Referenzspannung $W_{Ref}$ zugeführt.

Bei der beschriebenen Auslegung der Schaltung des Teils C nimmt der Komparator 74 an seinem Ausgang hohes Potential an, sobald die Körperoberflächenspannung weniger als 3 % der Ausgangsspannung des HF-Chirurgiegerätes beträgt. Von dem Komparator 74 wird das Relais 78 angesteuert, das über den Kontakt 80 das Elektrochirurgiegerät 1 abschaltet. Gleichzeitig schaltet das Relais 78 über seine Schaltkontakte den Signalgeber 79 ein. Der Signalgeber 79 zeigt optisch und/oder akustisch an, daß die Kontrollelektrode 4 nicht mehr ordnungsgemäß sitzt und deshalb eine Überprüfung notwendig ist.

Der Teil C der Überwachungsschaltung ermöglicht es schließlich, die Gefahren zu erkennen und automatisch auszuschließen, die bei fehlender Neutralelektrode oder bei ungenügendem Kontakt der Neutralelektrode auftreten. Hat nämlich die neutrale Elektrode 3 keinen oder nur ungenügenden Kontakt zur Körperoberfläche, dann besteht die Gefahr, daß auf der Körperoberfläche hohe Spannungsspitzen entstehen, die zu Funkenüberschlägen zwischen dem Körper und der Neutralelektrode und infolgedessen zu entsprechenden Verletzungen führen können.

Wie bei den voraufgehend beschriebenen Teilen der Überwachungsschaltung wird auch im Teil D die von der Kontrollelektrode 4 abgegriffene Körperoberflächenspannung mit Hilfe eines aus den beiden Widerständen 81 und 82 bestehenden Spannungsteilers auf den hundertsten Teil heruntergeteilt. Die so reduzierte Spannung wird wiederum einem HF-Umformer 83 zugeführt, der in diesem Fall als Spitzenwert-Detektor ausgebildet ist, wie er anhand der Fig. 5 beschrieben wird. Die von dem HF-Umformer 83 gelieferte Gleichspannung wird dem Plus-Eingang des Komparators 84 zugeleitet. Als Referenzspannung dient in diesem Fall eine konstante Gleichspannung von 1,5 Volt. Auf diese Weise wird von dem Komparator 84 das Relais 85 angesteuert, sobald die Körperoberflächenspannung einen Wert von 150 Volt erreicht oder überschreitet. Das Relais 85 schaltet über seinen Kontakt 86 das Elektrochirurgiegerät 1 ab. Die ebenfalls betätigten Kontakte 87 des Relais 85 schalten einen optischen und/oder akustischen Signalgeber 89 ein, durch den angezeigt wird, daß die Neutralelektrode nicht oder zumindest nicht ordnungsgemäß angelegt ist.

Der Aufbau eines für die Anwendung in der Überwachungsschaltung geeigneten Spitzenwert-Detektors als HF-Umformer, beispielsweise im Fall der HF-Umformer 73, 77 und 83, wird anhand der Fig. 5 im einzelnen beschrieben.

Über den Koppelkondensator 91 wird die HF-Spannung $U_{Ein}$ dem Transistor 92 zugeführt. Wenn die Spannung $U_{Ein}$ größer als 0,5 V ist, arbeitet der Transistor 92 als Emitterfolger auf den Emitterwiderstand 93. Der Kollektorstrom des Transistors 92 erzeugt im Potentiometer 94 einen Spannungsabfall, mit dem der Basisstrom des Transistors 95 und damit auch sein Kollektorstrom einreguliert werden kann. Ein Teil des Kollektorstroms des Transistors 95 durchfließt die Diode 96 und den Transistor 97, der wie eine versteilerte Diode arbeitet. Die Diode 96 und der Transistor 97 haben die Aufgabe, das Potential gegenüber dem Potential des Emitterwiderstandes 93 um zwei Schleusenspannungen zu erhöhen. Der andere Teil des Kollektorstroms vom Transistor 95 fließt über die Diode 98 und lädt den Kondensator 99 auf. Da der Ladevorgang über den Transistor 95 und die Diode 98 sehr schnell erfolgt, der Entladevorgang über den Widerstand 100 jedoch langsam vor sich geht, wird sich nach wenigen positiven Halbwellen der eingehenden HF-Spannung $U_{Ein}$ der Kondensator 99 auf eine Ausgangsspannung $U_{Aus}$ aufgeladen haben, die dem Spitzenwert der Eingangsspannung $U_{Ein}$ entspricht. Mit Hilfe des Potentiometers 94 kann man den Spitzenwert-Detektor so einstellen, daß einem Spitzenwert der Eingangsspannung $U_{Ein}$ von 1 V eine Ausgangsspannung $U_{Aus}$ von ebenfalls 1 V entspricht. Die Zeitkonstante von Kondensator 99 und Widerstand 100 wird so gewählt, daß die Ausgangsspannung $U_{Aus}$ lange genug ansteht, um an dem nachfolgenden Spannungskomparator mit der Referenzspannung verglichen werden zu können. Wenn der Spannungskomparator beispielsweise im 0,1 ms-Bereich arbeitet, dann genügt eine Zeitkonstante von beispielsweise 2 ms für den aus dem Kondensator 99 und dem Widerstand 100 bestehenden Kreis.

Bei den beschriebenen Ausführungsbeispielen ist die Überwachungsschaltung so ausgeführt, daß in den Spannungskomparatoren Analogsignale verarbeitet werden. Es ist selbstverständlich auch möglich, die Schaltung in Digitaltechnik auszuführen und einen Mikroprozessor zur Verarbeitung der Signale einzusetzen.

**Ansprüche**

**1.** Überwachungsvorrichtung für ein Hochfrequenz-Chirurgiegerät (1), mit einer in größerer Entfernung zur aktiven Elektrode (2) am Körper angebrachten, während der Behandlung die auf der Körperoberfläche bestehende auf das Potential der neutralen Elektrode bezogene Hochfrequenzspannung abgreifenden ' Kontrollelektrode (4) und mit einer von der Kontrollelektrode angesteuerten, einen Spannungskomparator (12; 54) umfassenden Überwachungsschaltung, wobei der Spannungskomparator eine von der Körperoberflächenspannung abhängige Spannung mit einer vorgegebenen Referenzspannung ($U_{REF}$) vergleicht und bei Überschreiten der Referenzspannung einen Signalgeber und/oder ein das Hochfrequenz-Chirurgiegerät abschaltendes Relais (16) ansteuert, **dadurch gekennzeichnet,** daß dem Spannungskomparator (12; 54) einerseits eine der von der Kontrollelektrode (4) abgegriffenen Spannung direkt proportionale Spannung, und andererseits als Referenzspannung ($U_{Ref}$) eine einer zuvor bei jedem Patienten individuell ermittelten maximal erträglichen Körperoberflächenspannung entsprechende Spannung zugeführt wird.

**2.** Überwachungsvorrichtung nach Anspruch 1, dadurch gekennzeichnet, daß zur individuellen Bestimmung der im Einzelfall maximal erträglichen Körperoberflächenspannung eine zwei in einem festen Abstand ($D_A$) voneinander angeordnete Kontaktelektroden (23, 24) und einen eine einstellbare Hochfrequenzspannung liefernden HF-Generator (31) umfassende Einrichtung vorgesehen ist.

**3.** Überwachungsvorrichtung nach Anspruch 2, dadurch gekennzeichnet, daß der Abstand ($D_A$) der beiden Meßelektroden (23, 24) voneinander etwa das Zehnfache des Durchmessers (d) der Meßelektroden (23, 24) beträgt.

**4.** Überwachungsvorrichtung nach Anspruch 2 und 3, dadurch gekennzeichnet, daß an einer der beiden Meßelektroden (24) ein die Erwärmung der Haut messender Temperaturfühler (25) angeordnet ist.

**5.** Überwachungsvorrichtung nach einem der Ansprüche 1 bis 4, dadurch gekennzeichnet, daß die Überwachungsschaltung zur Überprüfung des ordnungsgemäßen Kontaktes der neutralen Elektrode (3) einen weiteren Spannungskomparator (64) umfaßt, dem einerseits eine der von der Kontrollelektrode (4) abgegriffenen Spannung direkt proportionale Spannung, und andererseits als Referenzspannung ($V_{Ref}$) eine zu der Arbeitsspannung direkt proportionale Spannung zugeführt wird, wobei das Verhältnis der dem Komparator (64) zugeführten Spannungen so gewählt ist, daß der Spannungskomparator (64) ein Signal gibt, wenn die von der Kontrollelektrode (4) abgegriffene Körperoberflächenspannung einen Wert von etwa 10 % der Arbeitsspannung übersteigt.

**6.** Überwachungsvorrichtung nach einem der Ansprüche 1 bis 5, dadurch gekennzeichnet, daß die Überwachungsschaltung zur Überprüfung des Kontaktes der Kontrollelektrode (4) einen weiteren Spannungskomparator (74) umfaßt, dem einerseits eine zu der von der Kontrollelektrode (4) abgegriffenen Spannung direkt proportionale Spannung, und andererseits als Referenzspannung ($W_{Ref}$) eine zu der Arbeitsspannung direkt proportionale Spannung zugeführt wird, wobei das Verhältnis der Spannungen so gewählt wird, daß der Spannungskomparator (74) ein Signal gibt, wenn die von der Kontrollelektrode (4) abgegriffene Spannung kleiner ist als etwa 3 % der Arbeitsspannung.

**7.** Überwachungsvorrichtung nach einem der Ansprüche 1 bis 6, dadurch gekennzeichnet, daß die Überwachungsschaltung zur Überprüfung schädlicher Spannungsspitzen einen weiteren Spannungskomparator (84) umfaßt, der ein Signal gibt, wenn die von der Kontrollelektrode (4) abgegriffene Körperoberflächenspannung einen Wert von etwa 150 Volt übersteigt.

**8.** Überwachungsvorrichtung nach einem der Ansprüche 1 bis 7, dadurch gekennzeichnet, daß das von dem Spannungskomparator (12; 54) gelieferte Signal ein Schaltrelais (16; 56) ansteuert, das das Hochfrequenz-Chirurgiegerät (1) für eine Zeitspanne ausschaltet, die für eine Abkühlung der Körperteile unter der neutralen Elektrode (3) ausreicht.

**9.** Überwachungsvorrichtung nach Anspruch 5, dadurch gekennzeichnet, daß das von dem Spannungskomparator (64) gelieferte Signal ein Schaltrelais (68) ansteuert, durch das ein Licht- und/oder Tonsignal (69) eingeschaltet wird.

**10.** Überwachungsvorrichtung nach Anspruch 6, dadurch gekennzeichnet, daß das von dem Spannungskomparator (74) gelieferte Signal ein Schaltrelais (78) ansteuert, durch das das Hochfrequenz-Chirurgiegerät (1) abgeschaltet und ein Licht- und/oder Tonsignal (79) eingeschaltet wird.

**11.** Überwachungsvorrichtung nach Anspruch 7, dadurch gekennzeichnet, daß das von dem Spannungskomparator (84) gelieferte Signal ein Schaltrelais (85) ansteuert, durch das das Hochfrequenz-Chirurgiegerät (1) abgeschaltet und ein Licht- und/oder Tonsignal (89) eingeschaltet wird.

**12.** Überwachungsvorrichtung nach einem der Ansprüche 1 bis 11, dadurch gekennzeichnet, daß zur Umwandlung der von der Kontrollelektrode (4) auf der Körperoberfläche abgegriffenen Hochfrequenzspannung in eine den Spannungskomparatoren (12; 54, 64, 74, 84) zugeführte dem Effektivwert der Hochfrequenzspannung entsprechende Gleichspannung linearisierte Thermoumformer verwendet werden.

**13.** Überwachungsvorrichtung nach Anspruch 12, dadurch gekennzeichnet, daß die linearisierten Thermoumformer jeweils aus zwei gleichen Thermoumformern (38, 41) bestehen, deren Thermoelemente (40, 43) in Subtraktionsschaltung an die Eingänge eines Operationsverstärkers (45) gelegt sind, wobei die auf den Heizwiderstand (42) des Thermoumformers (41) gegebene Ausgangsspannung des Operationsverstärkers (45) dem nachgeschalteten Komparator zugeführt wird.

**14.** Überwachungsvorrichtung nach einem der Ansprüche 1 bis 11, dadurch gekennzeichnet, daß zur Umwandlung der von der Kontrollelektrode (4) auf der Körperoberfläche abgegriffenen Hochfrequenzspannung in eine den Spannungskomparatoren zugeführte Gleichspannung eine einen Kondensator (99) aufladende Emitter-Folger-Schaltung verwendet wird, bei der die Ausgangsspannung des Kondensators (99) gleich dem Spitzenwert der Eingangsspannung wird.

**15.** Überwachungsvorrichtung nach Anspruch 14, dadurch gekennzeichnet, daß die Emitter-Folger-Schaltung einen von der Kontrollelektrode (4) über einen Spannungsteiler angesteuerten, als Emitterfolger auf den Emitterwiderstand (93) arbeitenden Transistor (92), ein einen Spannungsabfall des Kollektorstroms des Transistors (92) erzeugendes Potentiometer (94) sowie einen Transistor (95) umfaßt, dessen Basis mit dem Abgriff des Potentiometers (94), und dessen Kollektor einerseits mit einer aus einer Diode (96) und einem sich daran anschließenden Transistor (97) bestehenden, das Potential gegenüber dem Potential des Emitterwiderstandes (93) um zwei Schleusenspannungen erhöhenden Schaltung, und andererseits über eine Diode (98) mit dem Kondensator (99) verbunden ist, zu dem ein Entladewiderstand (100) parallel geschaltet ist.

**16.** Überwachungsvorrichtung nach Anspruch 15, dadurch gekennzeichnet, daß die Zeitkonstante des aus dem Kondensator (99) und dem Widerstand (100) bestehenden Kreises auf den nachfolgenden Komparator abgestimmt und so gewählt ist, daß die zu dem Komparator führende Ausgangsspannung im Komparator den Vergleich mit der entsprechenden Referenzspannung erlaubt.

## Claims

**1.** Monitoring device for a high-frequency surgical appliance (1), with a control electrode (4), which is mounted at the body at a greater distance from the active electrode (2) and during the treatment taps off the high-frequency voltage existing on the surface of the body and referred to the potential of the neutral electrode, and with a monitoring circuit driven by the control electrode and comprising a voltage comparator (12; 54), wherein the voltage comparator compares a voltage dependent on the body surface voltage with a preset reference voltage ($U_{Ref}$) and, on the reference voltage being exceeded, drives a signal transmitter and/or a relay (16) switching off the high-frequency surgical appliance, characterised thereby, that on the one hand, a voltage directly proportional to the voltage tapped off by the control electrode (4) and, on the other hand as reference voltage ($V_{Ref}$), a voltage corresponding to the maximum tolerable body surface voltage previously determined individually for each patient is fed to the voltage comparator (12; 54).

**2.** Monitoring device according to claim 1, characterised thereby, that an equipment which comprises two contact electrodes (23, 24) arranged each at a fixed spacing ($D_A$) from the other and a high-frequency generator (31) supplying a settable high-frequency voltage, is provided for the individual determination of the maximum body surface voltage tolerable in the individual case.

**3.** Monitoring device according to claim 2, characterised thereby, that the spacing ($D_A$) of both the measuring electrodes (23, 24) each from the other amounts to about ten times the diameter (d) of the measuring electrodes (23, 24).

4. Monitoring device according to claim 2 and 3, characterised thereby, that a temperature sensor (15), which measures the heating of the skin, is arranged at one of both the measuring electrodes (24).

5. Monitoring device according to one of the claims 1 to 4, characterised thereby, that the monitoring circuit for the checking of the orderly contact of the neutral electrode (3) comprises a further voltage comparator (64), to which is fed on the one hand a voltage directly proportional to the voltage tapped off by the control electrode (4) and, on the other hand as reference voltage ($U_{Ref}$), a voltage directly proportional to the operating voltage, wherein the ratio of the voltages fed to the comparator (64) is so chosen that the voltage comparator (64) delivers a signal when the body surface voltage tapped off by the control electrode (4) exceeds a value of about 10% of the operating voltage.

6. Monitoring device according to one of the claims 1 to 5, characterised thereby, that the monitoring circuit for the checking of the contact of the control electrode (4) comprises a further voltage comparator (74), to which is fed on the one hand a voltage directly proportional to the voltage tapped off by the control electrode (4) and, on the other hand as reference voltage ($U_{Ref}$), a voltage directly proportional to the operating voltage, wherein the ratio of the voltages is so chosen that the voltage comparator (74) delivers a signal when the voltage tapped off by the control electrode (4) is smaller than about 3% of the operating voltage.

7. Monitoring device according to one of the claims 1 to 6, characterised thereby, that the monitoring circuit for the checking of harmful voltage peaks comprises a further voltage comparator (84), which delivers a signal when the body surface voltage tapped off by the control electrode (4) exceeds a value of about 150 volts.

8. Monitoring device according to one of the claims 1 to 7, characterised thereby, that the signal delivered by the voltage comparator (12; 54) drives a switching relay (16; 56), which switches the high-frequency surgical appliance (1) off for a time span which suffices for a cooling-down of the body parts under the neutral electrode (3).

9. Monitoring device according to claim 5, characterised thereby, that the signal delivered by the voltage comparator (64) drives a switching relay (68), through which a light signal and/or a sound signal (69) is switched on.

10. Monitoring device according to claim 6, characterised thereby, that the signal delivered by the voltage comparator (74) drives a switching relay (78), through which the high-frequency surgical appliance (1) is switched off and a light signal and/or a sound signal (79) is switched on.

11. Monitoring device according to claim 7, characterised thereby, that the signal delivered by the voltage comparator (84) drives a switching relay (85), through which the high-frequency surgical appliance (1) is switched off and a light signal and/or a sound signal (89) is switched on.

12. Monitoring device according to one of the claims 1 to 11, characterised thereby, that linearised thermal converters are used for the conversion of the high-frequency voltage tapped off at the body surface by the control electrode (4) into a direct voltage which corresponds to the effective value of the high-frequency voltage and is fed to the voltage comparators (12; 54, 64, 74, 84).

13. Monitoring device according to claim 12, characterised thereby, that the linearised thermal converters each consist of two like thermal converters (38, 41), the thermocouples (40, 43) of which are applied in subtractive connection to the inputs of an operational amplifier (45), wherein the output voltage of the operational amplifier (45) is applied to the heating resistor (42) of the thermal converter (41) and fed to the comparator connected therebehind.

14. Monitoring device according to one of the claims 1 to 11, characterised thereby, that an emitter follower circuit, which charges a capacitor (99) and in which the output voltage of the capacitor (99) is equal to the peak value of the input voltage, is used for the conversion of the high-frequency voltage tapped off at the body surface by the control electrode (4) into a direct voltage fed to the voltage comparators.

15. Monitoring device according to claim 14, characterised thereby, that the emitter follower circuit comprises a first transistor (92), which is driven by the control electrode (4) by way of a voltage divider and works as emitter follower on the emitter resistance (93), a potentiometer (94) producing a voltage drop of the collector current of the transistor (92) as well as a transistor (95), the base of which is connected with the tap of the potentiometer (94) and the collector of which is connected on the one hand with a circuit, which consists of a diode (96) and a transistor (97) connected thereto and which increases the potential relative to the potential of the emitter resistance (93) by two switching voltages, and on the other hand by way of a diode (98) with the capacitor (99), with which a discharge resistor (100) is connected in parallel.

**16.** Monitoring device according to claim 15, characterised thereby, that the time constant of the circuit, which consists of the capacitor (99) and the resistor (100), is matched to the following comparator and so chosen that the ouput voltage leading to the comparator permits the comparison with the corresponding reference voltage in the comparator.

## Revendications

**1.** Dispositif de surveillance pour un appareil chirurgical à haute fréquence (1), comportant une électrode de contrôle (4) disposée sur le corps à grande distance de l'électrode active (2), et relevant, pendant l'opération, la tension à haute fréquence, rapportée au potentiel de l'électrode neutre et existant à la surface externe du corps, et comportant un circuit de surveillance, commandé par l'électrode de contrôle et comprenant un comparateur de tension (12, 54), étant entendu que le comparateur de tension compare une tension fonction de la tension de la surface externe du corps, avec une tension de référence ($U_{Ref}$) donnée au préalable et commande, en cas de dépassement de la tension de référence, un émetteur de signal et/ou un relais (16) mettant hors circuit l'appareil chirurgical à haute fréquence, caractérisé en ce qu'on envoie au comparateur de tension (12, 54), d'une part, une tension directement proportionnelle à la tension relevée par l'électrode de contrôle (4), et, d'autre part, comme tension de référence ($U_{Ref}$), une tension correspondant à la tension maximale supportable par la surface externe du corps, déterminée au préalable dans le cas de chaque patient.

**2.** Dispositif de surveillance suivant la revendication 1, caractérisé en ce que, pour la détermination, par individu, de la tension maximale supportable sur la surface externe du corps dans son cas particulier, il est prévu une installation comprenant deux électrodes de contact (23, 24), disposés à une distance fixe ($D_A$) l'une de l'autre, et un générateur HF (31) délivrant une tension haute fréquence réglable.

**3.** Dispositif de surveillance suivant la revendication 2, caractérisé en ce que la distance ($D_A$), séparant les deux électrodes de mesure (23, 24) l'une de l'autre, est environ dix fois le diamètre (d) des électrodes de mesure (23, 24).

**4.** Dispositif de surveillance suivant la revendication 2 et la revendication 3, caractérisé en ce que, sur l'une des deux électrodes de mesure (24), est disposée une sonde de température (25) mesurant l'échauffement de la peau.

**5.** Dispositif de surveillance suivant l'une quelconque des revendications 1 à 4, caractérisé en ce que le circuit de surveillance pour contrôler que le contact de l'électrode neutre (3) est en ordre, comporte un autre comparateur de tension (64) auquel sont envoyées, d'une part, une tension directement proportionnelle à la tension relevée par l'électrode de contrôle (4) et, d'autre part, comme tension de référence ($V_{Ref}$), une tension directement proportionnelle à la tension de travail, le rapport des tensions arrivant au comparateur (64) étant choisi de telle façon que le comparateur de tension (64) émet un signal si la tension sur la surface externe du corps relevée par l'électrode de contrôle (4) dépasse une valeur d'environ 10 % de la tension de travail.

**6.** Dispositif de surveillance suivant l'une quelconque des revendications 1 à 5, caractérisé en ce que le circuit de surveillance pour contrôler le contact de l'électrode de contrôle (4), comporte un autre comparateur de tension (74) auquel sont envoyées, d'une part, une tension directement proportionnelle à la tension relevée par l'électrode de contrôle (4) et, d'autre part, comme tension de référence ($W_{Ref}$) une tension directement proportionnelle à la tension de travail, le rapport des tensions étant choisi de telle façon que le comparateur de tension (74) émet un signal si la tension relevée par l'électrode de contrôle (4) est inférieure à environ 3% de la tension de travail.

**7.** Dispositif de surveillance suivant l'une quelconque des revendications 1 à 6, caractérisé en ce que le circuit de surveillance, pour contrôler des pointes de tension dangereuses, comporte un autre comparateur de tension (84) qui émet un signal si la tension sur la surface externe du corps relevée par l'électrode de contrôle (4) dépasse une valeur d'environ 150 Volt.

**8.** Dispositif de surveillance suivant l'une quelconque des revendications 1 à 7, caractérisé en ce que le signal délivré par le comparateur de tension (12; 54) commande un relais de coupure (16; 56) qui déclenche l'appareil électrochirurgical à haute fréquence (1) pour une fourchette de temps suffisante pour un refroidissement des parties du corps sous l'électrode neutre (3).

**9.** Dispositif de surveillance suivant la revendication 5, caractérisé en ce que le signal délivré par le comparateur de tension (64) commande un relais de coupure (68) qui enclenche un signal lumineux et/ou sonore (69).

**10.** Dispositif de surveillance suivant la revendication 6, caractérisé en ce que le signal délivré par le comparateur de tension (74) commande un relais de coupure (78) qui débranche l'appareil chirurgical à haute fréquence (1) et qui enclenche un signal lumineux et/ou sonore (79).

**11.** Dispositif de surveillance suivant la revendication 7, caractérisé en ce que le signal délivré par le comparateur de tension (84) commande un relais de coupure (85) qui débranche l'appareil chirurgical à haute fréquence (1) et qui enclenche un signal lumineux et/ou sonore (89).

**12.** Dispositif de surveillance suivant l'une quelconque des revendications 1 à 11, caractérisé en ce que, pour la transformation de la tension haute fréquence, relevée par l'électrode de contrôle (4) sur la surface externe du corps, en une tension continue correspondant à la valeur efficace de la tension haute fréquence envoyée aux comparateurs de tension (12; 54, 64, 74, 84), on utilise des thermoconvertisseurs linéarisés.

**13.** Dispositif de surveillance suivant la revendication 12, caractérisé en ce que les thermoconvertisseurs linéarisés sont chacun constitués de deux thermoconvertisseurs linéarisés identiques (38, 41) dont les thermoéléments (40, 43) en montage soustractif sont placés aux entrées d'un amplificateur opérationnel (45), la tension de sortie de l'amplificateur opérationnel (45) donnée sur la résistance de chauffage (42) du thermoconvertisseur (41) étant envoyée au comparateur branché à la suite.

**14.** Dispositif de surveillance suivant l'une quelconque des revendications 1 à 11, caractérisé en ce que, pour la transformation de la tension haute fréquence, relevée par l'électrode de contrôle (4) sur la surface externe du corps, en une tension continue envoyée aux comparateurs de tension, on utilise un montage émetteur-suiveur chargeant un condensateur (99), montage pour lequel la tension de sortie du condensateur (99) devient égale à la valeur de pointe de la tension d'entrée.

**15.** Dispositif de surveillance suivant la revendication 14, caractérisé en ce que le montage émetteur-suiveur comprend un transistor (92), travaillant comme émetteur-suiveur sur la résistance d'émetteur (93) et commandé par l'électrode de contrôle (4) au moyen d'un diviseur de tension, un potentiomètre (94) créant une chute de tension du courant du collecteur du transistor (92), ainsi qu'un transistor (95) dont la base est reliée avec la sortie du potentiomètre (94), et dont le collecteur est relié, d'une part, avec un montage qui releve le potentiel de deux tensions de seuil par rapport au potentiel de la résistance d'émetteur (93) et qui est constitué d'une diode (96) et d'un transistor (97) qui lui fait suite, et dont la base est reliée, d'autre part, par l'intermédiaire d'une diode (98), avec le condensateur (99), auquel est raccordée en parallèle une résistance de décharge (100).

**16.** Dispositif de surveillance suivant la revendication 15, caractérisé en ce que la constante de temps du circuit constitué du condensateur (99) et de la résistance de décharge (100) est accordée sur le comparateur qui fait suite et est choisie de telle façon que, dans le comparateur, la tension de sortie arrivant au comparateur permet la comparaison avec la tension de référence correspondante.

3
4
2
1
6
Fig. 1
17
18
19
7
9
8
11
12
10
13
16
U_Ref
U_=
14
20
30
32
31
T
34
V
33
28
28
29
29
28
28
29
29
22
26
26
27
27
23
d
D_A
24
25
Fig. 2

Fig. 4
4
2
3
1
6
52
86
80
57
58
51
53
54
56
$U_{Ref}$
55
ZU WARM
59
A
62
61
63
64
67
$V_{Ref}$
65
66
68
KONTAKT-
NEUTRAL-
ELEKTRODE
69
B
72
71
$W_{Ref}$
80
73
77
74
75
76
78
KONTAKT-
KONTROLL-
ELEKTRODE
79
C
82
81
83
84
85
86
87
87
1,5V
NEUTRAL-
ELEKTRODE
FEHLT
89
D

9
I1
38
I2
41
40
39
43
42
V
45
11
46

Fig. 3

UBat
94
95
91
98
96
UEin
UAus
92
97
99
100
93

Fig. 5